# EUROPEAN PATENT APPLICATION

(11) **EP 4 548 968 A1**
(43) Date of publication of application: **07.05.2025**
(21) Application number: 24213933.5
(22) Date of filing: 19.11.2024
(51) Int. Cl.: A61N 5/10

(54) **IRRADIATION DEVICE AND METHOD FOR SCANNING A PREDETERMINED TARGET AREA WITH A PULSED PARTICLE BEAM**

(71) Applicant: Deutsches Elektronen-Synchrotron DESY, 22607 Hamburg (DE); Karlsruher Institut für Technologie, 76131 Karlsruhe (DE)
(72) Inventor: SACK, Martin, 76287 Rheinstetten (DE); HERZOG, Dennis, 76676 Graben-Neudorf (DE); LOISCH, Gregor, 20535 Hamburg (DE); OBIER, Frank, 24582 Bordesholm (DE); STEPHAN, Frank, 15732 Eichwalde (DE)
(74) Representative: Seemann & Partner Patentanwälte mbB

(57) **Abstract**

The invention relates to an irradiation device (1) and a method for scanning a predetermined target area (10) with a pulsed particle beam (20). The irradiation device (1) comprises a radiation source (2) and a deflection device (3). The radiation source (2) is configured to emit the pulsed particle beam (20) comprising a plurality of pulse trains (22), each pulse train (22) comprising a plurality of pulses (24). Each pulse train (22) has a length of 1 µs to 1ms. The irradiation device (1) further comprises a pulse power source (4) configured to supply a deflection voltage (50) to the deflection device (3), wherein the deflection voltage (50) comprises multiple stepped voltage pulses (51, 52) each comprising multiple voltage steps (53, 54). The stepped voltage pulses (51, 52) are synchronized to the pulse trains (22) in order to scan the predetermined target area (10).

## Description

The invention relates to an irradiation device. The invention further relates to a method for scanning a predetermined target area with a pulsed particle beam.

Currently, radiation therapy of tumors is mostly carried out with photons or ions. In most cases, an electron accelerator is used to generate the photons. This electron accelerator can in principle also be used to supply electrons to radiate the tissue. However, electrons have a higher energy output in the near-surface layers of the tissue compared to photons. This is why typically photon irradiation results in less radiation damage to healthy tissue compared to electron irradiation, when employing current treatment methods for treating deep-seated tumors.

However, this can be compensated by the so called Flash effect. When applying the Flash effect, the dosage is increased from typically 0.03 Gy/s to values greater than 40 Gy/s and the irradiation time is often reduced to less than 100 ms. It has been shown that irradiation at this very high dose rate and short duration damages tumor tissue at least as much as conventional irradiation, but at the same time the surrounding healthy tissue is spared much better. Thus, the Flash effect makes irradiation with electrons more relevant for clinical use.

To avoid unnecessary irradiation of healthy tissue around the tumor, the transverse extent of the electron beam should not be larger than the transverse shape of the tumor. This could be achieved, for example, by creating a mask that absorbs those parts of the electron beam that lie outside the desired area. However, such a mask has to be created individually for specific tumor shapes and has to meet certain requirements in term of its material and secondary irradiation. There are also flexible, mechanical masks, so called Multi-leaf masks. However, the adjustment of these masks is relatively slow. In addition, the absorption of the unwanted electrons via the mask creates additional radiation, which requires additional shielding.

As an alternative approach, the transverse extent of the electron beam may be chosen to be significantly smaller than the area to be irradiated. However, in this case it is necessary for the electron beam to scan the desired area by moving the electron beam across the area. In order to do so, the electron beam has to be deflected in order to change its path.

The deflection of particle packages poses a technical challenge for the beam guidance elements, also known as deflection devices, which direct the particle beam from the accelerator to the tumor to be irradiated. These deflection devices must be able to distribute the particles of a pulse train within a time of 1 ms or less, which is the typical maximum duration of a train of particle bunches from an electron accelerator for medical applications. In other words, since the typical high frequency pulse driving an electron accelerator is e.g. 1 ms or shorter, with a repetition rate of e.g. 10 Hz, the distribution must take place within a time of less than 1 ms.

It is the object of the present invention to provide an irradiation device, in particular for radiation therapy, and a method for scanning a predetermined area with the pulsed particle beam, which are able to scan the predetermined area with the particle beam within a time of 1 ms or less.

This object is achieved by an irradiation device, in particular for radiation therapy, comprising a radiation source and a deflection device, wherein the radiation source is configured to emit a pulsed particle beam comprising a plurality of pulse trains, each pulse train comprising a plurality of pulses, wherein each pulse train has a length of at least 1 µs, characterized by a pulse power source configured to supply a deflection voltage to the deflection device, wherein the deflection voltage comprises multiple stepped voltage pulses each comprising multiple voltage steps, wherein the stepped voltage pulses of the deflection voltage are synchronized to the pulse trains of the particle beam to scan a predetermined target area.

By supplying the deflection device with a deflection voltage from a pulse power source, wherein the deflection voltage comprises multiple stepped voltage processes which are synchronized to the pulse trains of the particle beam, the deflection device is able to deflect each pulse train of the particle beam individually and distribute the radiation of the particle beam on the predetermined target area.

The predetermined target area is, for example, a tumor to be irradiated. The predetermined target area may also be a sample to be analysed. A pulse spacing of the stepped voltage pulses supplied by the pulse power source is, in particular, synchronized with the pulse spacing of the pulse trains of the particle beam. The pulse spacing is the spacing between each pulse train of the particle beams and/or between each stepped voltage pulse of the deflection voltage, respectively. In particular, each pulse train has a length of 1 µs to 1 ms. The pulse power source is part of the irradiation device.

The deflection voltage may also be a deflection electrical power. Thus, the pulse power source may be configured to supply the deflection electrical power to the deflection device. In this case, the deflection electrical power comprises multiple stepped power pulses each comprising multiple power steps. However, with a static impedance, a drop of the deflection voltage leads to a drop of a deflection current.

The stepped voltage pulse comprises multiple voltage steps. A voltage value of the deflection voltage usually changes between subsequent voltage steps. However, if a certain area is supposed to be irradiated by multiple bunches of the radiation, the deflection voltage may remain constant in subsequent steps. The deflection device is designed such that there is a functional relationship, in particular a linear relationship, between the deflection voltage and the deflection of the particle beam. Thus, when the voltage value of the deflection voltage changes, so does the deflection intensity of the particle beam. By choosing appropriate voltage values for each voltage step, the particle beam can be deflected such that it scans the predetermined target area.

The irradiation device may comprise two deflection devices, which are configured to deflect the particle beam in orthogonal directions. The irradiation device may alternatively comprise a deflection device configured to deflect the particle beam in two orthogonal directions. This allows the irradiation device to scan a predetermined target area, whose length and width are both larger than the extend of the particle beam.

Preferably, the voltage steps of the stepped voltage pulses are synchronized to the pulses of the pulse trains. Thus, each pulse in each pulse train of the particle beam can be individually directed towards a certain point or region in the predetermined target area. In this way, a specific dose rate can be allocated to each part of the predetermined target area. This allows the irradiation device to adjust the dose rate and the duration of the irradiation easily, so as to best employ the Flash effect for radiation therapy.

Even when the voltage steps are synchronized with the pulses of the pulse trains, this does not mean that all voltage steps have to have the same length as the pulses. The length of the voltage steps may be a multiple or a fraction of the length of the pulses, as long as they are synchronized.

Preferably, a duration of the voltage pulses is 1 µs to 1 ms. In particular, the voltage pulses have a similar length to the pulse trains. The length of the voltage pulses may be essentially equal to the length of the pulse trains.

According to an embodiment, at least one voltage step of the the stepped voltage pulses is set to a voltage value equal to n times U0, where n is an integer and U0 is a constant voltage value. n may be a natural number, a negative number or zero. In particular, multiple voltage steps of the the stepped voltage pulses are set to a voltage value equal to n times U0. Further in particular, each voltage step of the the stepped voltage pulses is set to a voltage value equal to n times U0. In particular, multiple voltage steps of the the stepped voltage pulses are set to voltage values equal to nᵢ times U0 with i being the i-th step in a sequence of s steps (1 <= i <= s). Further in particular, for each voltage step i of the stepped voltage pulses, the voltage is set to a voltage value equal to nᵢ times U0. In particular, nᵢ is chosen individually for each step, independent of the selection for any other step. Hence, the amplitude of any individual voltage step can be selected arbitrarily. This makes it possible to deflect consecutive pulses in an equidistant fashion, resulting in a uniform irradiation of the target area, by increasing or decreasing nᵢ by one for each consecutive step i, resulting in a staircase-like pattern of consecutive voltage steps.

Preferably, the pulse power source comprises a main control device and a plurality of step modules each comprising a local microcontroller, wherein the main control device is configured to generate a clock signal for each voltage step of the stepped voltage pulses and transmit the clock signal to each step module, wherein the step modules are configured, when receiving the clock signal, to emit a voltage according to a local switching plan stored on a local memory of each step module, wherein the voltages generated by each step module combine to form a voltage value of each voltage step of the stepped voltage pulses. By using a plurality of step modules to create individual voltage parts, which are then combined to the voltage value of each voltage step, the voltage value of each voltage step can be individually adjusted. By providing each step module with a local microcontroller, it is possible to change the voltage output of each step module in a very short time frame. In order to further accelerate the process, a local switching plan is stored on a local memory of each step module. This local switching plan is provided to the step module in advance and indicates, which voltage the step module emits at each step of a voltage pulse. In order to synchronize the output of each step module, they are provided with the clock signal from the control device. In summary this setup of the pulse power source provides a very fast switching time, which allows the allocation of individual pulses of a pulse train to different locations within the predetermined target area. The step modules comprise in particular one or more parallel bridge circuits, in particular formed of semiconductor switches, for example SiC-MOSFETs. Each step module comprises in particular a capacitor battery and a charger. By implementing a separate battery and charger for each step module, the step modules can all be charged eaxactly to the desired value, for example U0..

Preferably, the plurality of step modules comprise a main group of step modules, wherein the step modules of the main group are configured to emit a voltage equal to +U0 or -U0 or 0 when receiving the clock signal, wherein in particular the plurality of step modules comprise at least one supplemental group of step modules, wherein the step modules of the supplemental group are configured to emit a voltage equal to +U1 or -U1 or 0 when receiving the clock signal, wherein the voltage value of U1 is different from U0. By triggering a number of step modules of the main group, a voltage step with a voltage value equal to n times U0 may be generated. The step module or modules of the supplemental group make it possible to generate different voltage values. For example, the value of U0 may be double the value of U1. Thus, by triggering the step modules of the supplemental group in addition to triggering the step modules of the main group, it is possible to generate finer step values.

Preferably, the voltage steps comprise a first voltage step and a directly subsequent second voltage step, wherein the main control device is configured to control the step modules in such a way, that the second voltage step is generated by as many step modules as possible that were unused for generating the first voltage step. By using step modules that were unused in the generation of the previous step, the individual step modules are discharged equally. In particular, the step modules used for the second step are those step modules, which have the highest charge value. This also allows the step modules to receive additional charging time.

Preferably, the step modules are connected in series. By connecting the step modules in series, the voltage generated by each step module adds up to form the voltage value of each voltage step.

Preferably, each voltage step of the stepped voltage pulses has a duration of less than 1 ms, in particular less than 1 µs, further in particular less than 100 ns. By decreasing the duration of each voltage step, the rate at which the deflection of the particle beam can be changed, is increased. By using voltage steps with such small durations, it is possible to scan the predetermined target area within the time of a single pulse train, even if the pulse train has a very short length.

Preferably, an energy of the particle beam is between 1 keV and 10 GeV, in particular between 1MeV and 300 MeV.

Preferably, the radiation source is configured to emit a pulsed electron beam or a pulsed ion beam as the pulsed particle beam. Thus, the pulsed particle beam may be an electron beam or an ion beam. The ion beam may comprise H-nuclei, carbon ions or similar ions.

According to an embodiment, the irradiation device comprises two pulse power sources configured to supply two separate deflection voltages to two separate deflection units, wherein the two deflection units deflect the particle beam in orthogonal directions. By supplying two separate deflection voltages to two separate deflection units, the particle beam can be deflected in two orthogonal directions, allowing the particle beam to scan a two-dimensional area. The irradiation device may comprise two deflection devices each with one deflection unit or one deflection device with two deflection units. The two deflection units may comprise a horizontal deflection unit and vertical deflection unit.

According to an embodiment, the deflection device comprises a kicker magnet with two independent electrodes, wherein the kicker magnet is in particular a stripline kicker. A kicker magnet is advantageous for the irradiation device, as it allows the deflection of pulse trains of very short pulse separation. The deflection device comprises in particular two orthogonal kicker magnets or a kicker magnet with four electrodes, two of which are orthogonal to the other two. The stripline kicker comprises, in particular, at least two striplines to deflect the particle beam. In particular, the stripline kicker comprises four striplines, two for each direction. As an alternative to a stripline kicker, the kicker magnet may comprise at least one inductive conductor loop.

The object is further achieved by a method for scanning a predetermined target area with a pulsed particle beam, wherein a radiation source emits the pulsed particle beam comprising a plurality of pulse trains, each pulse train comprising a plurality of pulses, wherein each pulse train has a length of at least 1 µs, characterized by that a pulse power source supplies a deflection voltage to a deflection device, wherein the deflection voltage comprises multiple stepped voltage pulses each comprising multiple voltage steps, wherein the stepped voltage pulses of the deflection voltage are synchronized to the pulse trains of the particle beam to scan the predetermined target area.

The method embodies the same advantages, features and properties as the previously described irradiation device.

Preferably, the voltage steps of the stepped voltage pulses are synchronized to the pulses of the pulse trains.

Preferably, a duration of the voltage pulses is 1 µs to 1 ms.

The irradiation device may implement a computer-based method for a combined feed-back control of the individual stage voltages and the adjustable signal propagation delays between the main control unit and the stages' individual voltage pulses at the pulse generator's output terminal. The method comprises the repeated sequence of the following process steps for every n-th pulse with n preferably between 1 and 10000:
(1) Measurement and digital acquisition of a) a stepped voltage pulse at the pulse generator's output terminal combined with a time signal from the main control unit that marks the point in time at which the main control unit sends the signal to switch to the following step to the stages, and b) the initial voltages across the stage capacitors at the individual stages just before the pulse generation.
(2) Extraction of the pulse voltage at points in time at the end of each time step, preferably, just before the moment of switching when the main control unit sends the time signal to switch to the next step.
(3) Checking whether the amplitudes of the extracted pulse voltages (see step 2) are well within the acceptable ranges. If yes, continue with step 8.
(4) Setup of a linear equation system describing the dependency of the extracted pulse voltages (see step 2) at the end of each time step, *M,* of the initial voltages across the stage capacitors at the individual stages *U₀,* in dependence of the switch matrix S describing the varying configuration of active and passive stages for each considered step, the matrix R containing the internal resistances of each individual stage at each time step depending on the stages' switch configurations in relation to the constant load resistance of the pulse generator, and the matrix ***U_{Q}*** describing the time integral of the pulse voltage over the active time of a stage only, weighted with the value of the stage capacitance individually for each stage and time step as follows: *M = **S*** · *U₀* - **R** · *M* - ***U_{Q}*** · *1*
(5) Solving the equation system to obtain an estimation for U*₀*. The equation system is rendered to be solvable by an appropriate selection of included steps or groups of steps.
(6) Comparison of the estimated values for *U₀* with the initially measured values for *U₀*.
(7) Linear adjustment of the individual initial charging values of the stages.
(8) Extraction of the points in time, when the slope of each voltage change between two adjacent steps of the stepped voltage pulse at the generator's output crosses the two levels of preferably at least +/-10 % of the value of the charging voltage per stage *U₀* with respect to the steady-state level of the previous step and with respect to the steady-state level of the following step.
(9) For each point in time defining the begin of a voltage transition between two steps the difference in time to the corresponding time signal from the main control unit is calculated and compared to a setpoint value, which needs to be at least as long as the longest signal travelling time from the main control unit to the generator's output through any stage. By selecting a larger value, an additional signal delay can be implemented. If the calculated time difference deviates by more than +/- 1ns from the setpoint values, the time delay settings of all active stages of the following step are adjusted equally.
(10) Approximation of the slope of each voltage change between two steps of the stepped voltage pulse at the generator's output by means of a straight through the voltage-time coordinates at the two levels identified in step 8.
(11) Check whether the rise times of the approximated slopes are faster than a defined limit for the rise time. Such a limit is defined by the selected type of semiconductor switches and the circuit design. Values may range preferably between 0.1 ns and 1 µs. If yes, then proceed with step 13.
(12) For each transition comprising more than one switching stage whereas the delay time of all but one stage has already been adjusted either in step 9 of the current run of the sequence or in one of the recent runs the delay time of the stage of unknown delay time is modified such that the slope fits the requirements.
(13) The identification numbers of the stages for which the delay time has been determined within the current sequence are stored for the procedure described in step 12 for a number of runs at maximum equal to the total number of generator stages.
(14) If the current switching pattern does not allow to determine and adjust the initial charging voltages U₀ and delay times of all stages within one run of the sequence, the switching patterns of stages are modified such that the stages are swapped against each other in the sequence of steps between subsequent runs of this sequence whereas the shape of the pulse voltage is not modified.

Further characteristics of the invention will become apparent from the description of the embodiments according to the invention together with the claims and the included drawings. Embodiments according to the invention can fulfill individual characteristics or a combination of several characteristics.

The invention is described below, without restricting the general intent of the invention, based on exemplary embodiments, wherein reference is made expressly to the drawings with regard to the disclosure of all details according to the invention that are not explained in greater detail in the text. The drawings show in:
- Fig. 1: a schematic simplified representation of an irradiation device, in particular for radiation therapy,
- Fig 2: a schematic simplified representation of a deflection device,
- Fig. 3: a schematic simplified representation of a horizontal and a vertical deflection voltage supplied by pulse power sources to the deflection device,
- Fig 4.: a schematic simplified representation of a particle beam with multiple pulse trains each comprising multiple pulses and a deflection voltage,
- Fig. 5: a schematic simplified representation of a pulse power source,
- Fig. 6: a schematic simplified representation of a first embodiment of a deflection device with kicker magnets,
- Fig. 7: a schematic simplified representation of a second embodiment of a kicker magnet,
- Fig. 8a to 8e: schematic simplified cross sections of kicker magnets with differently shaped stripline kickers,
- Fig.9: a schematic simplified representation of a deflection voltage with sixteen steps, and
- Fig. 10: a listing of the step modules used to generate each of the sixteen steps shown in Fig. 9.

In the drawings, the same or similar types of elements or respectively corresponding parts are provided with the same reference numbers in order to prevent the item from needing to be reintroduced.

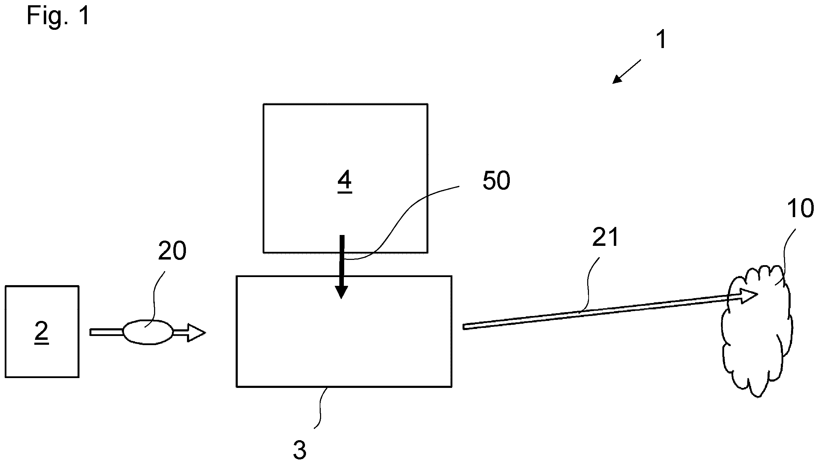

Fig. 1 shows a schematic simplified representation of an irradiation device 1 for radiation therapy. The irradiation device 1 comprises a radiation source 2, a deflection device 3 and a pulse power source 4. The radiation source 2 emits a pulsed particle beam 20, for example an electron beam. The pulsed particle beam 20 passes through the deflection device 3, which deflects the particle beam 20 through electromagnetic force. The deflected particle beam 21 is directed towards a predetermined target area 10. The pulse power source 4 is connected to the deflection device 3 and supplies a deflection voltage 50 to the deflection device 3. By controlling and changing the voltage value of the deflection voltage 50, the amount by which the particle beam 20 is deflected can be controlled. In this way, the entire predetermined target area 10 can be scanned by the particle beam 20.

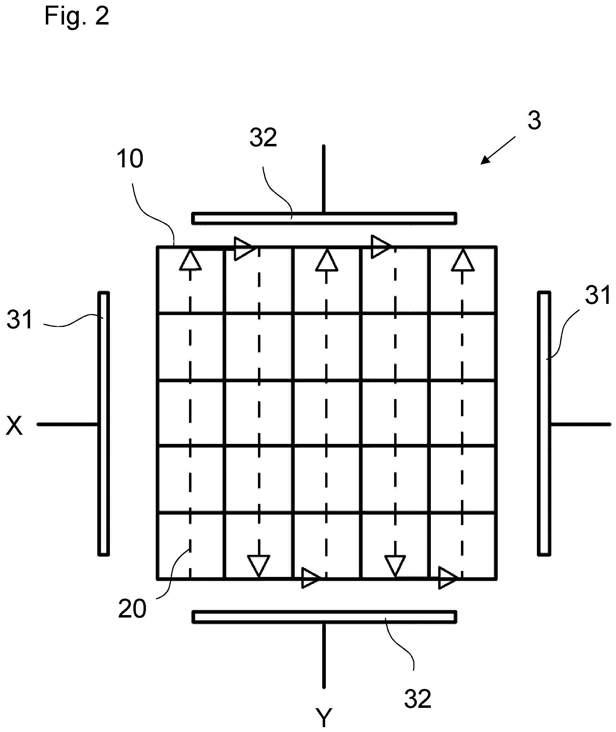

Fig. 2 shows a schematic simplified representation of an embodiment of the deflection device 3 as well as the predetermined target area 10. The deflection device 3 in this embodiment comprises two deflection units 31, 32, which each comprises two elements, for example electrodes. The deflection unit 31 is a horizontal deflection unit, while the deflection unit 32 is a vertical deflection unit. Each of the deflection units 31, 32 is supplied with a separate deflection voltage 50. By controlling the rate at which the deflection voltage 50 supplied to each deflection unit 31, 32 changes, it is possible to deflect the particle beam 20 in a way that it scans the entire target area 10. This is done by first changing the vertical deflection voltage 50 stepwise from a lowest value to a highest value, then increasing the horizontal deflection voltage 50 by a single step, then lowering the vertical reflection voltage 50 again stepwise and so on until the entire target area 10 is scanned.

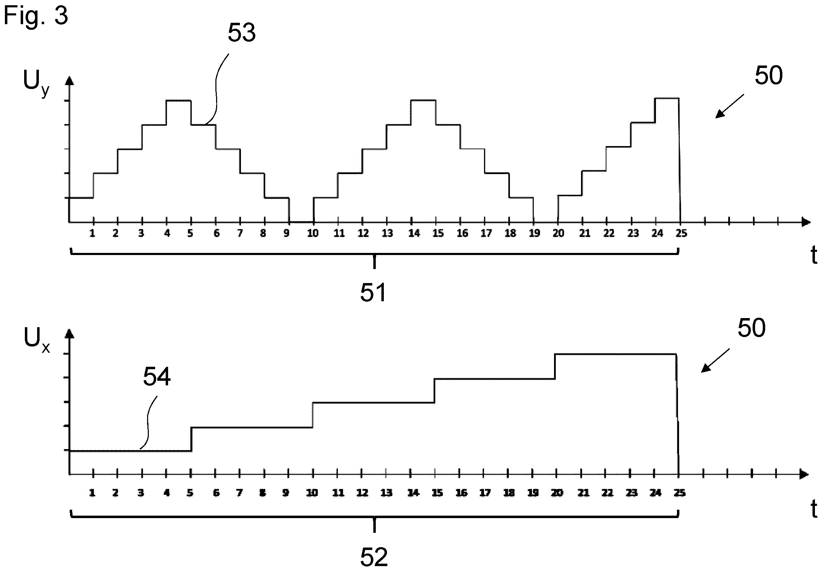

Fig. 3 shows a schematic representation of the curves for the vertical deflection voltage 50 (Uy) and the horizontal defection voltage 50 (Ux). As can be seen in Fig. 3, the deflection voltage 50 comprises stepped voltage pulses 51, 52 with multiple voltage steps 53, 54. For sake of clarity, only one of the voltage steps 53 in Uy and one of the steps 54 in Ux is assigned a reference number. Uy is first increased stepwise over time t until a maximum is reached and Ux is increased as well. Then Uy is decreased stepwise again until a minimum is reached and Ux is increased another step. This is repeated until the entire target area 10 has been evenly irradiated.

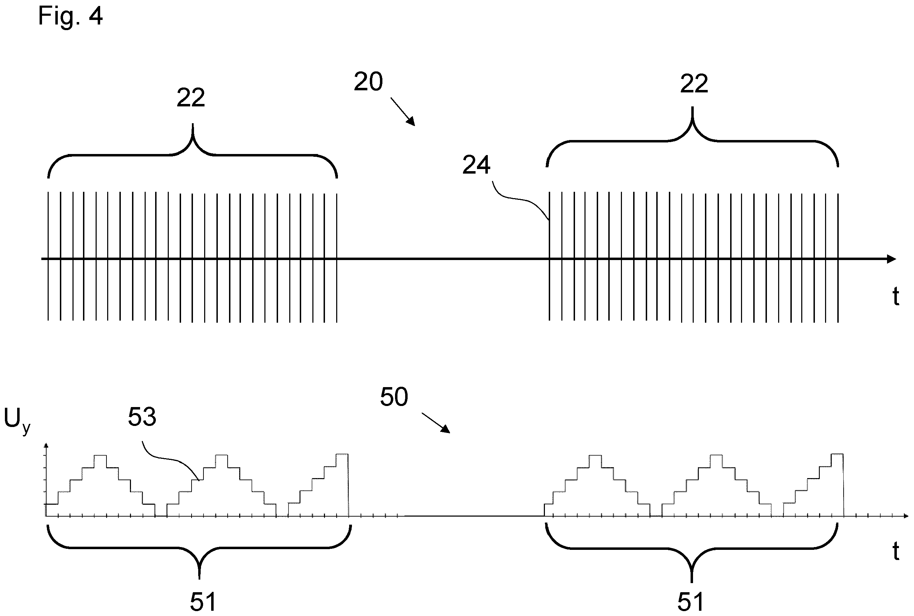

Fig. 4 shows a schematic simplified representation of a particle beam 20 with a plurality of pulse trains 22, each comprising multiple pulses 24. For sake of clarity, only one of the pulses 24 is assigned a reference number. A typical duration of a pulse train 22 is about 10 µs to 1 ms. The repetition rate of the pulse trains 22 may be about 10 Hz, so that a spacing between pulse trains 22 is about 100 ms. Thus, for sake of clarity, the two pulse trains 22 shown in Fig. 4 are shown to be much closer together than they would normally be. The distance between two pulses may be 1 µs to 0.3 ns, depending on the radiation source 2.

In order to deflect each individual pulse train 22, the stepped voltage pulses 51, 52 are synchronized to the pulse trains 22 as shown in Fig. 4. This allows each pulse train 22 to be deflected individually and identically, causing a uniform irradiation of the target area 10. In this way, the dose rate and irradiation time can be specifically tailored to the desired values to individually treat each patient.

As shown in Fig. 4, each pulse 24 may also be synchronized to each voltage step 53, 54 of the deflection voltage 50. In this way, each pulse 24 may be distributed to a different area of the predetermined target area 10 and a uniform irradiation is achieved. However, for very short distances between pulses 24, each step 53, 54 of the deflection voltage 50 may correspond to multiple pulses 24 of the particle beam 20. In this case, the distance between the pulses 24 is small enough and the charge of the pulses 24 low enough that a smooth transition between each step 53, 54 will be achieved nonetheless. In this case, the deflection voltage 50 has to remain constant for long enough that a sufficient dose is deposited at each position of the target area 10.

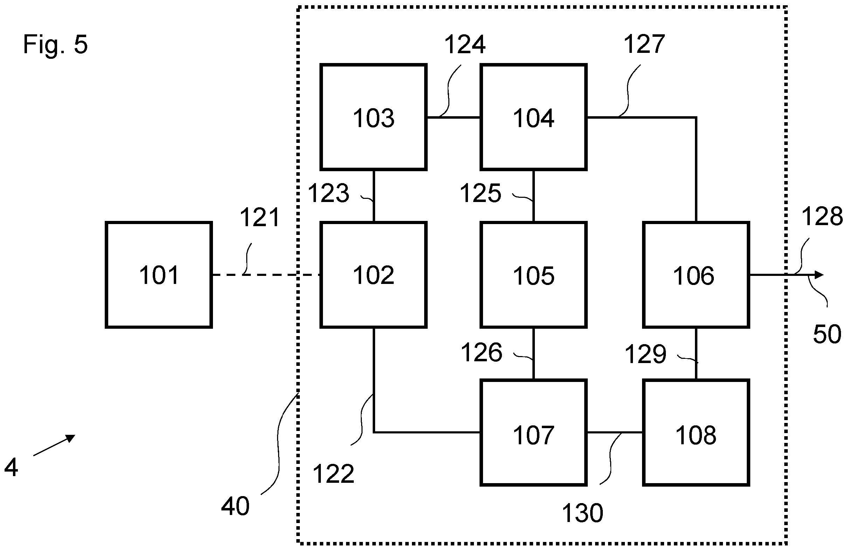

Fig. 5 shows a schematic simplified representation of a pulse power source 4 with a step module 40. The pulse power source 4 comprises a main control device 101 and multiple step modules 40. However, for simplicity sake, only a single step module 40 is shown in Fig. 5. Typically, each pulse power source 4 may comprise eight or more step modules 40.

The main control device 101 is connected via a fibre optic cable 121 to a transmitting and receiving unit 102 of each step module 40. The main control device 101 sends a clock signal 123 through the fibre optic cable 121 to the transmitting and receiving unit 102, which transmits the clock signal 123 to a time delay element 103. The time delay element 103 compensates the time differences of the clock signal 123 to the different step modules 40 in order to ensure that the clock signal 123 is processed simultaneously in all step modules 40. The delayed clock signal 124 is transmitted to a flow control 104. The flow control 104 reads a local memory 105 through storage connection 125 and provides control signals 127 to a pulse voltage source 106. The control signals can either be +U0, -U0 or 0, with U0 being a constant voltage value. Thus, the pulse voltage source 106 is controlled to emit either a voltage of +U0 or -U0 or 0, when receiving the signal from the flow control 104. This voltage is emitted by a pulse exit 128. As the multiple step modules 40 are arranged in series, the voltage values of the step modules 40 add up to form the individual voltage steps 53, 54 of the step voltage pulses 51, 52.

The step module 40 also comprises a microcontroller 107, which is connected to the transmitting and receiving unit 102 via data transmission 122. The microcontroller 107 also has a storage connection 126 to the local memory 105 to save a local switching plan on the local memory 105. The local switching plan is specific for each step module 40 and dictates for each voltage step 53, 54 if the step module 40 emits a voltage of +U0, -U0 or 0. By storing the local switching plan on the local memory 105 in advance, the amount of information that has to be sent from the main control device 101 to each step module 40 is greatly reduced, which accelerates the switching time and the minimal step duration. The microcontroller 107 is also connected to a charging circuit 108 via a control cable 130. The charging circuit 108 is adjustable and provides a charging voltage 129 to the pulse voltage source 106.

The interaction of several step modules 40, each parameterized with an individual switching program, creates the desired voltage curve of the deflection voltage 50. The common clock signal 123 is provided to each step module 40 to ensure that the steps are advanced synchronously.

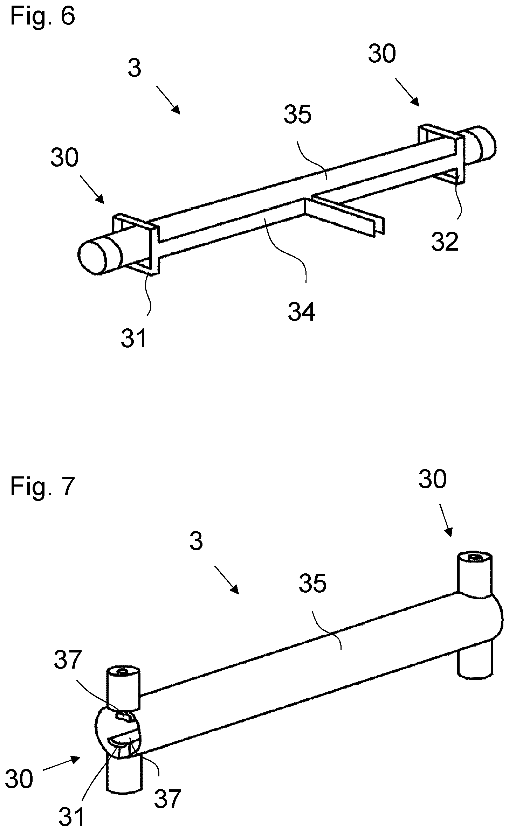

Figs. 6 and 7 show different embodiments of a deflection device 3. Fig. 6 shows a deflection device 3 with a kicker magnet 30 arranged on the outside of a tube 35, through which the particle beam 20 is guided. The deflection device 3 comprises two kicker magnets 30, one for horizontal and one for vertical deflection. This is realized through two deflection units 31, 32 configured as inductive conductor loops 34, which create a magnetic field that deflects the particle beam 20.

Fig. 7 shows a different type of kicker magnet 30. This kicker magnet 30 comprises stripline kickers 37 that extend along the longitudinal direction of the tube 35. At both ends of the tube 35, the stripline kickers 37 are provided with connections, which feed the stripline kickers 37 with electrical energy. The stripline kickers 37 create an electromagnetic field in-between them that deflects the particle beam 20 running through the tube 35. In the exemplary embodiment shown in Fig. 7 the kicker magnet 30 comprises only two stripline kickers 37 that form a single deflection unit 31 to deflect the particle beam 20 in a single direction. In order to also deflect the particle beam 20 in an orthogonal direction, additional stripline kickers 37 are required.

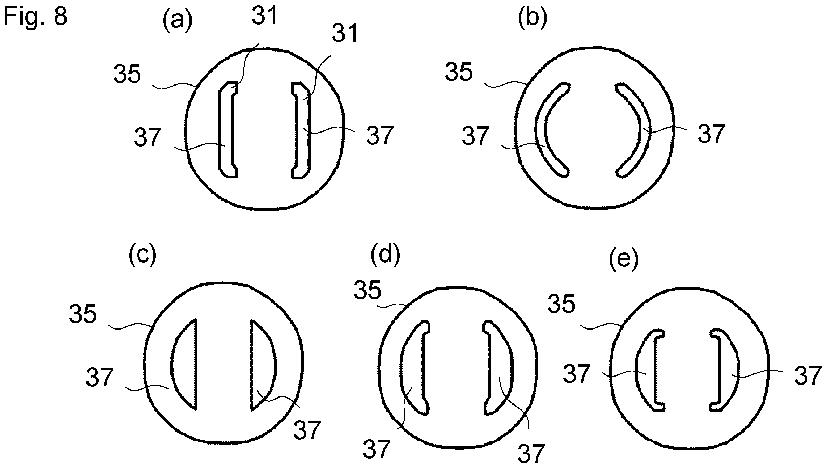

Figs. 8a to 8e show different embodiments of kicker magnets 30, which comprise stripline kickers 37 with different cross sections. In Fig. 8a, the cross section of the stripline kickers 37 is almost straight. In Fig. 8b, the cross section has a form of two circular arcs and in Fig. 8c, the form of two circular segments. The stripline kickers 37 in Figs. 8d and 8e are mixed forms of the other examples.

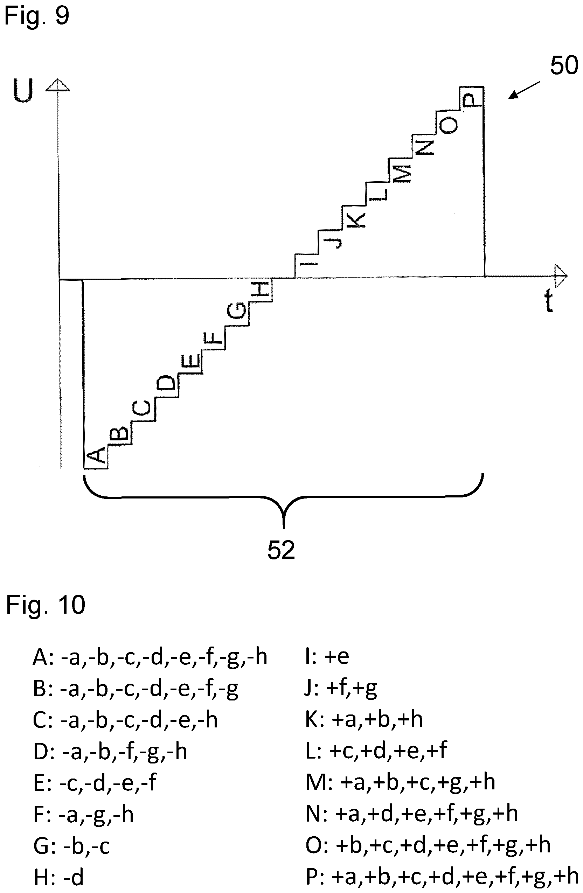

Fig. 9 and 10 explain the triggering of the different step modules 40 in order to create the voltage steps 53, 54. As can be seen in Fig. 9, an exemplary deflection voltage 50 has a total of sixteen steps which are assigned the upper case letters A to P. The steps A to H have negative voltage values, the steps I to P positive voltage values. In this example, each consecutive step has the same height due to the fact that each step module generates a voltage of +U0, -U0 or 0. However, it is also possible to create steps with different heights by using step modules from a supplemental group, which may generate voltages of +U1, -U1 or 0, with U1 different from U0.

In order to create the steps A to P, a total of eight step modules 40 are triggered, which are assigned the lower case letters a to h in the listing shown in Fig. 10. Fig. 10 shows for each step A to P if a step module 40 generates a positive voltage U0, a negative voltage U0 or zero voltage. For example, for step A all eight step modules 40 generate a negative voltage, whereas for the step P all eight step modules 40 generate a positive voltage. When possible, different step modules 40 are triggered in subsequent steps. For example, in step E, the step modules c, d, e and f are triggered, whereas in the subsequent step F, the step modules a, g and h are triggered. This allows the step modules a to h to be charged and discharged equally.

A voltage drop across the semiconductor switches occurs depending on the current. It is intended to supply a matched load with a known and constant load impedance, for example 50 ohms. The ratio of voltage and current at the load is therefore known and enables a compensation of the voltage drops, especially of the voltage across the temperature-dependent internal impedance of each individual semiconductor switch (i.e. for a MOSFET the value R_{DS,on}) in the following ways:
a) the individual stage voltage U0ₘ of each module m is adjusted such that the absolute value of the module's output voltage stays within a predefined tolerance band when considering the voltage drops across the impedances R_{DS,on} of the module's conducting MOSFET switches and the due to the discharging or charging process varying voltage of the module's capacitor. If the pulse repetition rate is sufficiently low, e.g. 10 Hz, the semiconductor switches have time to cool down significantly, so that the internal impedance (e.g. R_{DS,on} ) is lower than after a longer period of time while conducting a significantly high current. If the capacitor of one module is discharged during pulse generation, both effects are superimposed and the module's output voltage drops accordingly with the time.
b) The pulse generator's output voltage is measured, e.g. by means of a voltage divider coupled to an oscilloscope, and compared to the sum of voltages of the capacitors of all active modules, i.e. modules delivering a voltage different from zero in any individual step. Thereby, all voltages are acquired at the same moment of time. If only one module is active during the measurement, the voltage drop can be calculated as difference between the voltage across the active module's capacitor and the output voltage. If more than one module is active, the individual voltage drops can be calculated based on m measurements, if m is the total number of active modules in a series of i steps. The voltage drops at each individual module can be calculated by solving a linear equation system, provided, that the combination of active modules in the course of the m measurements is linearly independent in the mathematical sense. If the individual voltage drops of each module are known, the modules' charging voltages U0ᵢ can be adjusted to compensate for the voltage drop and, hence, adapt the modules to varying environmental conditions, e.g. varying ambient temperature.

During signal transmission via fibre optic cables from the generator's main control unit to the individual generator modules differences in the individual propagation delay times occur due to different lengths of the fibre optic cables or unequal and time-variable attenuation of the light signal, e.g. due to bending of the fibre optic cables. According to the invention, these differences in propagation delay times are equalised by adding an adjustable delay module into each signal path, either as part of the main control unit or preferably as part of each individual stage module, which can be adjusted individually for each stage module to compensate for unequal delay times. This is done on the basis of individual measurements of the switching times of individual stage modules by determining the individual time difference between the generation of the switching signal in the main control unit and the change in the measured output voltage for each stage module. This works particularly well if only one or a few stages are active in a switching step. By comparing the measured delay times at different steps and configurations of active modules, the delay times for each stage module can be calculated, provided that the combinations of active modules is selected appropriately, i.e. allows for the solution of a linear equation system. For this evaluation the same measurement setup for the output voltage can be employed as for checking the voltage amplitude as described above. In addition to or as an alternative to determining the switching times during pulse generation, the switching times can also be determined before pulse generation or between two pulses by switching the stage modules individually or in groups especially for such measurements, either one time for calibration purpose or repeatedly in order to adapt the delays to changing conditions. Moreover, an over-all delay of the pulse at the generator's output versus the generator's input signal can be performed by means of adding an appropriate offset value to all delay modules.

All named characteristics, including those taken from the drawings alone, and individual characteristics, which are disclosed in combination with other characteristics, are considered alone and in combination as important to the invention. Embodiments according to the invention can be fulfilled through individual characteristics or a combination of several characteristics. Features which are combined with the wording "in particular" or "especially" are to be treated as preferred embodiments.

### List of References

- 1: irradiation device
- 2: radiation source
- 3: deflection device
- 4: pulse power source
- 10: predetermined target area
- 20: particle beam
- 21: deflected particle beam
- 22: pulse train
- 24: pulse
- 30: kicker magnet
- 31, 32: deflection unit
- 34: inductive conductor loop
- 35: tube
- 37: stripline kicker
- 40: step module
- 50: deflection voltage
- 51, 52: voltage pulse
- 53, 54: voltage step
- 101: main control device
- 102: transmitting and receiving unit
- 103: time delay element
- 104: flow control
- 105: local memory
- 106: pulse voltage source
- 107: microcontroller
- 108: charging circuit
- 121: fibre optic cable
- 122: data transmission
- 123: clock signal
- 124: delayed clock signal

- 125: storage connection (reading)
- 126: storage connection (writing)
- 127: control signals (+U0, -U0, 0)
- 128: pulse exit
- 129: charging voltage
- 130: control cable

- A to P: step
- a to h: step module

## Claims

1. Irradiation device (1), in particular for radiation therapy, comprising a radiation source (2) and a deflection device (3), wherein the radiation source (2) is configured to emit a pulsed particle beam (20) comprising a plurality of pulse trains (22), each pulse train (22) comprising a plurality of pulses (24), wherein each pulse train (22) has a length of at least 1 µs, **characterized by** a pulse power source (4) configured to supply a deflection voltage (50) to the deflection device (3), wherein the deflection voltage (50) comprises multiple stepped voltage pulses (51, 52) each comprising multiple voltage steps (53, 54), wherein the stepped voltage pulses (51, 52) of the deflection voltage (50) are synchronized to the pulse trains (22) of the particle beam (20) to scan a predetermined target area (10).

2. Irradition device (1) according to claim 1, wherein the voltage steps (53, 54) of the stepped voltage pulses (51, 52) are synchronized to the pulses (24) of the pulse trains (22).

3. Irradiation device (1) according to claim 1 or 2, wherein a duration of the voltage pulses (51, 52) is 1 µs to 1 ms.

4. Irradiation device (1) according to one of the claims 1 to 3, wherein at least one voltage step (53, 54) of the stepped voltage pulses (51, 52) is set to a voltage value equal to n times U0, where n is an integer and U0 is a constant voltage value.

5. Irradiation device (1) according to one of the claims 1 to 4, wherein the pulse power source (4) comprises a main control device (101) and a plurality of step modules (40) each comprising a local microcontroller (107), wherein the main control device (101) is configured to generate a clock signal (123) for each voltage step (53, 54) of the stepped voltage pulses (51, 52) and transmit the clock signal (123) to each step module (40), wherein the step modules (40) are configured, when receiving the clock signal (123), to emit a voltage according to a local switching plan stored on a local memory (105) of each step module (40), wherein the voltages generated by each step module (40) combine to form a voltage value of each voltage step (53, 54) of the stepped voltage pulses (51, 52).

6. Irradiation device according to claims 4 and 5, wherein the plurality of step modules (40) comprise a main group of step modules (40), wherein the step modules (40) of the main group are configured to emit a voltage equal to +U0 or -U0 or 0 when receiving the clock signal (123), wherein in particular the plurality of step modules (40) comprise at least one supplemental group of step modules (40), wherein the step modules (40) of the supplemental group are configured to emit a voltage equal to +U1 or -U1 or 0 when receiving the clock signal (123), wherein the voltage value of U1 is different from U0.

7. Irradiation device (1) according to claims 5 or 6, wherein the voltage steps (53, 54) comprise a first voltage step (E) and a directly subsequent second voltage step (F), wherein the main control device (101) is configured to control the step modules (40) in such a way, that the second voltage step (F) is generated by as many step modules (40) as possible that were unused for generating the first voltage step (E).

8. Irradiation device (1) according to one of the claims 5 to 7, wherein the step modules (40) are connected in series.

9. Irradiation device (1) according to one of the claims 1 to 8, wherein each voltage step (53, 54) of the stepped voltage pulses (51, 52) has a duration of less than 1 ms, in particular less than 1 µs, further in particular less than 100 ns.

10. Irradiation device (1) according to one of the claims 1 to 9, wherein the radiation source (2) is configured to emit a pulsed electron beam or a pulsed ion beam as the pulsed particle beam (20).

11. Irradiation device (1) according to one of the claim 1 to 10 comprising two pulse power sources (4) configured to supply two separate deflection voltages (50) to two separate deflection units (31, 32), wherein the two deflection units (31, 32) deflect the particle beam (20) in orthogonal directions.

12. Irradiation device (1) according to one of the claims 1 to 11, wherein the deflection device (3) comprises a kicker magnet (30) with two independent electrodes, wherein the kicker magnet is in particular a stripline kicker (37).

13. Method for scanning a predetermined target area (10) with a pulsed particle beam (20), wherein a radiation source (2) emits the pulsed particle beam (20) comprising a plurality of pulse trains (22), each pulse train comprising a plurality of pulses (24), wherein each pulse train (22) has a length of at least 1 µs, **characterized by** that a pulse power source (4) supplies a deflection voltage (50) to a deflection device (3), wherein the deflection voltage (50) comprises multiple stepped voltage pulses (51, 52) each comprising multiple voltage steps (53, 54), wherein the stepped voltage pulses (51, 52) of the deflection voltage (50) are synchronized to the pulse trains (22) of the particle beam (20) to scan the predetermined target area (10).

14. Method according to claim 13, wherein the voltage steps (53, 54) of the stepped voltage pulses (51, 52) are synchronized to the pulses (24) of the pulse trains (22).

15. Method according to claim 13 or 14, wherein a duration of the voltage pulses (51, 52) is 1 µs to 1 ms.
